# FASCICULE DE BREVET EUROPEEN

(11) **EP 0 987 017 B1**
(45) Date de publication et mention de la délivrance du brevet: **13.06.2001**
(21) Numéro de dépôt: 99402161.6
(22) Date de dépôt: 31.08.1999
(51) Int. Cl.: A61K 7/48

(54) **Compositions topiques cosmétiques ou dermatologiques comprenant des polyesters dendritiques**
Dendritische Polyester enthaltende kosmetische oder dermatologische topische Zusammensetzungen
Cosmetic or dermatologic topical compositions containing dendritic polyesters

(30) Priorité: 17.09.1998 FR 9811634
(43) Date de publication de la demande: 22.03.2000
(73) Titulaire: L'OREAL, 75008 Paris (FR)
(72) Inventeur: Tournilhac, Florence, 75012 Paris (FR); Simon, Pascal, 94400 Vitry/S/Seine (FR)
(74) Mandataire: Rasson, Catherine

(56) Documents cités:
- WO-A-93/17060

## Description

La présente invention concerne l'utilisation, dans des compositions topiques cosmétiques ou dermatologiques, d'un polymère dendritique de type polyester ou de l'association d'un tel polymère dendritique avec un polymère filmogène, et les compositions cosmétiques ou dermatologiques topiques comprenant ces polymères.

En cosmétologie et dermatologie il est intéressant de disposer de compositions qui, lorsqu'elles sont appliquées sur la peau, sont capables de former des films. Ces propriétés filmogènes ont pour avantages par exemple de favoriser la régularité des couches appliquées, de renforcer leur action adoucissante et protectrice de la peau, d'améliorer la qualité des dépôts de produits de maquillage tels que des fonds de teint ou des rouges à lèvres et de les rendre résistants à l'élimination par transfert (produits dits "sans transfert"), ou encore, dans le cas de masques ou de cataplasmes, de faciliter le nettoyage de la peau par simple pelage de la composition séchée.

Les composants apportant ces propriétés filmogènes sont généralement des polymères de haut poids moléculaire. L'approche consistant à augmenter la concentration de ces hauts polymères dans les compositions cosmétiques ou dermatologiques dans le but d'obtenir des propriétés filmogènes importantes n'est pas satisfaisante. Elle est en effet limitée par le fait que ces polymères augmentent trop fortement la viscosité du produit fini. Celui-ci devient trop épais, s'avère difficile à appliquer sur la peau et perd ses qualités sensorielles.

La demanderesse à découvert à présent qu'il était possible de surmonter ces inconvénients, c'est-à-dire de renforcer les propriétés filmogènes de compositions cosmétiques ou dermatologiques topiques sans nuire à leurs propriétés sensorielles, en utilisant un nouveau type de polymères appelés polymères dendritiques ou dendrimères ayant une structure polyester à groupements terminaux hydroxyle.

En effet, ces polymères dendritiques ou dendrimères décrits plus en détail ci-après, bien que très peu visqueux, sont capables notamment de renforcer la cohésion d'un film formé par des polymères filmogènes habituels. Leur incorporation dans des formulations cosmétiques ou dermatologiques topiques n'a pas d'effets néfastes sur la texture ou les propriétés sensorielles de celles-ci.

La présente invention a donc pour objet une composition cosmétique ou dermatologique topique susceptible d'être appliquée sur la peau, les fibres kératiniques, les ongles, les semi-muqueuses et/ou les muqueuses, comprenant un polymère dendritique de type polyester à fonctions hydroxyle terminales, ou comprenant l'association d'un tel polymère dendritique de type polyester à fonctions hydroxyle terminales avec un polymère filmogène.

La présente invention a en outre pour objet un procédé de traitement cosmétique utilisant une composition cosmétique comprenant un polymère dendritique de type polyester à fonctions hydroxyle terminales, ou comprenant l'association d'un tel polymère dendritique de type polyester à fonctions hydroxyle terminales avec un polymère filmogène.

La présente invention a encore pour objet l'utilisation de la composition comprenant un polymère dendritique de type polyester à fonctions hydroxyle terminales, ou comprenant l'association d'un tel polymère dendritique de type polyester à fonctions hydroxyle terminales avec un polymère filmogène, pour la préparation d'une composition dermatologique destinée au traitement de la peau.

D'autres objets apparaîtront à la lecture de la description et des exemples qui suivront.

Les compositions selon l'invention trouvent une application particulièrement intéressante dans le domaine du soin et/ou du maquillage de la peau du visage et du corps, des fibres kératiniques, des ongles, des muqueuses et/ou des semi-muqueuses. On entend notamment par muqueuse la partie interne de la paupière inférieure, par semi-muqueuse les lèvres du visage et par fibres kératiniques notamment les cils, les sourcils et les cheveux.

Les compositions selon l'invention renfermant l'association d'un dendrimère et d'un polymère filmogène sont en particulier très performantes lorsqu'elles sont utilisées en tant que masques pelables appelés également masques "Peel Off". Il s'agit de gels, généralement à base de poly(alcool vinylique), qui sont étalés en couche épaisse sur la peau du visage ou du corps et sont retirés par simple pelage du film séché. Le pouvoir filmogène de l'association polymère filmogène-dendrimère assure alors une bonne cohésion au film qui se retire en un seul morceau et ne laisse pas de petits fragments à éliminer ultérieurement.

Les polymères dendritiques ou dendrimères (du grec *dendron =* arbre) sont des molécules polymères "arborescentes", c'est-à-dire très ramifiées inventées par D. A. Tomalia et son équipe au début des années 90 (Donald A. Tomalia *et al.*, *Angewandte Chemie, Int. Engl. Ed.,* vol. 29, n° 2, pages 138 - 175). Il s'agit de structures moléculaires construites autour d'un motif central généralement polyvalent. Autour de ce motif central sont enchaînés, en couches concentriques et selon une structure parfaitement déterminée, des motifs ramifiés d'allongement de chaîne donnant ainsi naissance à des macromolécules symétriques, monodispersées ayant une structure chimique et stéréochimique bien définie.

Les polymères dendritiques utilisés dans les compositions cosmétiques ou dermatologiques de la présente invention sont des dendrimères ayant la structure chimique d'un polyester et qui sont terminés par des groupements hydroxyle. La structure et la préparation de ces polymères est décrite dans la demande de brevet WO-A-93/17060.

Plus précisément, les polymères dendritiques utilisés dans les compositions de la présente invention peuvent être définis comme étant des macromolécules hautement ramifiées de type polyester, constituées
- d'un motif central dérivé d'un composé initiateur portant un ou plusieurs fonctions hydroxyle (a),
- de motifs d'allongement de chaîne dérivés d'une molécule d'allongement de chaîne portant une fonction carboxyle (b) et au moins deux fonctions hydroxyle (c),
chacune des fonctions hydroxyle (a) de la molécule centrale étant le point de départ d'une réaction de polycondensation (par estérification) qui débute par la réaction des fonctions hydroxyle (a) de la molécule centrale avec les fonctions carboxyle (b) des molécules d'allongement de la chaîne, puis se poursuit par réaction des fonctions carboxyle (b) avec les fonctions hydroxyle (c) des molécules d'allongement de la chaîne.

Le composé initiateur portant une ou plusieurs fonctions hydroxyle et formant le motif central autour duquel se construira la structure dendritique est un composé mono-, di- ou polyhydroxylé. Il est généralement choisi parmi
(a) un alcool monofonctionnel,
(b) un diol aliphatique, cycloaliphatique ou aromatique,
(c) un triol,
(d) un tétrol,
(e) un alcool de sucre,
(f) l'anhydro-ennéa-heptitol ou le dipentaérythritol,
(g) un α-alkylglycoside,
(h) un polymère polyalcoxylé obtenu par polyalcoxylation d'un des alcools (a) à (g), ayant une masse molaire au plus égale à 8000.

On peut citer à titre d'exemples de composés initiateurs préférés servant à préparer les polyesters dendritiques utilisés dans la présente invention le ditriméthylolpropane, le ditriméthyloléthane, le dipentaérythritol, le pentaérythritol, un pentaérythritol alcoxylé, le triméthyloléthane, le triméthylolpropane, un triméthylolpropane alcoxylé, le glycérol, le néopentylglycol, le diméthylolpropane ou le 1,3-dioxane-5,5-diméthanol.

On fait réagir sur ces composés inititateurs hydroxylés formant le motif central du futur dendrimère, des molécules dites molécules d'allongement de chaîne qui sont des composés de type diol-monoacide choisis parmi
- les acides monocarboxyliques comportant au moins deux fonctions hydroxyle, et
- les acides monocarboxyliques comportant au moins deux fonctions hydroxyle dont une ou plusieurs porte(nt) un substituant hydroxyalkyle.

Des exemples préférés de tels composés sont l'acide diméthylolpropionique, l'acide α,α-bis (hydroxyméthyl)-butyrique, l'acide α,α,α - tris(hydroxyméthyl)-acétique, l'acide α,α-bis(hydroxyméthyl)-valérique, l'acide α,α-bis(hydroxy)propionique et l'acide 3,5-dihydroxybenzoïque.

Dans un mode de réalisation particulièrement préféré de la présente invention, le composé initiateur est choisi parmi le ditriméthylolpropane, le triméthylolpropane, un pentaérythritol éthoxylé, le pentaérythritol ou le glycérol, et la molécule d'allongement de chaîne est l'acide diméthylolpropionique.

Les polymères dendritiques de type polyester à fonctions hydroxyle terminales utilisés dans les compositions de la présente invention sont caractérisés en outre par le fait qu'une partie des fonctions hydroxyle terminales du polymère dendritique de type polyester peuvent porter des substituants dérivés d'au moins un agent de terminaison de chaîne.

On utilise de préférence un polymère sans substituants dérivés. Toutefois, lorqu'une partie des fonctions hydroxyle terminales portent un substituant dérivé, la fraction de ces fonctions hydroxyle terminales portant un motif de terminaison de chaîne est généralement comprise entre 1 et 90 % en moles, de préférence entre 10 et 50 % en moles rapporté au nombre total de fonctions hydroxyle terminales.

Le choix approprié d'un agent de terminaison de chaîne approprié permet de modifier à souhait les propriétés physico-chimiques des polyesters dendritiques utilisés dans les compositions de la présente invention.

Ledit agent de terminaison de chaîne peut être choisi dans une grande variété de composés capables de former des liaisons covalentes avec les fonctions hydroxyle terminales. Ces composés englobent notamment
i) un acide monocarboxylique saturé ou un acide gras saturé ou un anhydride d'un tel composé,
ii) un acide gras insaturé,
iii) un acide monocarboxylique insaturé,
iv) un diisocyanate ou un oligomère d'un tel composé,
v) un produit d'addition préparé à partir d'un composé selon iv),
vi) un acide dicarboxylique ou polycarboxylique ou un anhydride d'un tel composé,
vii) un produit d'addition préparé à partir d'un composé selon vi),
viii) un acide monocarboxylique aromatique,
ix) une épihalogénhydrine,
x) un ester glycidylique d'un acide monocarboxylique ou d'un acide gras comportant de 1 à 24 atomes de carbone,
xi) un époxyde d'un acide gras insaturé comportant de 3 à 24 atomes de carbone.

Des composés de terminaison de chaîne préférés sont en particulier l'acide laurique, les acides gras de graines de lin, les acides gras de soja, les acides gras de suif, les acides gras d'huile de ricin déshydrogéné, l'acide caproïque, l'acide caprylique, le maléate de l'éther diallylique de triméthylolpropane, l'acide méthacrylique et l'acide acrylique.

Les polymères dendritiques de type polyester à fonctions hydroxyle terminales et portant éventuellement des groupements de terminaison de chaîne sont connus et sont commercialisés par la société PERSTORP.

Des polymères particulièrement préférés utilisés dans la présente invention sont
- un polyester dendritique obtenu par polycondensation d'acide diméthylolpropionique sur du triméthylolpropane, exempt d'agents de terminaison de chaîne commercialisé sous la dénomination BOLTORN H40 TMP CORE ;
- un polyester dendritique obtenu par polycondensation d'acide diméthylolpropionique sur du pentaérythritol polyoxyéthyléné (5 motifs d'OE sur chaque fonction hydroxyle), dont 50 % des fonctions hydroxyle sont estérifiées par de l'acide caprique/caprylique (nom technique HBP 3G estérifié) ;
- un polyester dendritique obtenu par polycondensation d'acide diméthylolpropionique sur du pentaérythritol polyoxyéthyléné (5 motifs d'OE sur chaque fonction hydroxyle), exempt d'agent de terminaison de chaîne (nom technique HBP Polyol 3G)
tous ces polymères étant des produits de la société PERSTORP.

Dans les compositions de la présente invention, les polymères dendritiques de type polyester à groupements terminaux hydroxyle peuvent être associés à des polymères filmogènes dans le but de renforcer le pouvoir filmogène de ceux-ci. On peut utiliser pour cela une variété de polymères filmogènes connus et physiologiquement acceptables.

Parmi les polymères filmogènes utilisables dans la présente invention, on peut citer les polymères synthétiques obtenus par polycondensation ou par polymérisation radicalaire, les polymères d'origine naturelle et des mélanges de ceux-ci.

On peut citer par exemple les polyuréthannes anioniques, cationiques, non ioniques ou amphotères, les polyuréthannes acryliques, les copolymères de type polyuréthanne-polyvinylpyrrolidone, les poly(ester uréthanne), les poly(éther uréthanne), les polyurées, les poly(urée uréthanne), les polyesters, les poly(ester amide), les polyesters à chaîne(s) grasse(s), les polyamides, les résines époxy-ester, les polymères et/ou copolymères acryliques et/ou vinyliques, les copolymères acryliques/silicones, les copolymères nitrocellulose/acryliques, les polymères d'origine naturelle éventuellement modifiés, les polymères résultant de la polymérisation radicalaire d'un ou de plusieurs monomères à l'intérieur et/ou partiellement en surface de particules préexistantes d'au moins un polymère choisi dans le groupe formé par les polyuréthannes, les polyurées, les polyesters, les poly(ester amide) et/ou les alkydes et des mélanges de ces polymères.

Dans un mode de réalisation préféré de l'invention, le polymère filmogène est le poly(acétate de vinyle) éventuellement partiellement ou totalement hydrolysé et en particulier le poly(alcool vinylique), autrement dit un poly(acétate de vinyle) hydrolysé à plus de 80 %. Un tel poly(alcool vinylique) préféré est commercialisé par exemple par la société AIR PRODUCTS sous la dénomination AIRVOL 540 (hydroxylé à 88 %).

Dans les compositions cosmétiques ou dermatologiques topiques de la présente invention contenant des dendrimères associés à des polymères filmogènes, le rapport en poids du polymère dendritique au polymère filmogène est compris dans l'intervalle allant de 1/100 à 1/1, et de préférence dans l'intervalle allant de 1/15 à 1/1.

Le polymère dendritique est présent dans les compositions de la présente invention à raison de 0,1 à 5 % en poids et de préférence à raison de 0,5 à 2 % en poids rapporté à la composition totale.

Les compositions topiques selon l'invention peuvent contenir en outre un ou plusieurs principes actifs cosmétiques ou dermatologiques, ou encore un ou plusieurs solvants ou adjuvants physiologiquement acceptables utilisés habituellement en cosmétique ou dermatologie.

Les principes actifs cosmétiques ou pharmaceutiques englobent par exemple les anti-inflammatoires, les anti-bactériens, les anti-fongiques, les anti-viraux, les anti-séborrhéiques, les anti-acnéiques, les kératolytiques, les anti-histaminiques, les anesthésiques, les agents cicatrisants, les modificateurs de la pigmentation, les filtres solaires, les agents anti-radicaux libres, les agents hydratants, les vitamines, les protéines, les céramides et autres composés similaires.

Les solvants que peuvent contenir les compositions cosmétiques ou dermatologiques de l'invention sont choisis parmi l'eau, les monoalcools inférieurs tels que l'éthanol ou l'isopropanol, les glycols tels que le diéthylèneglycol, les éthers de glycols tels que les alkyléthers d'éthylèneglycol ou de diéthylèneglycol, ou les esters d'acides gras, ces solvants étant utilisés seuls ou sous forme de mélange.

Les adjuvants physiologiquement acceptables sont choisis parmi les agents régulateur de pH, les agents anti-oxydants, les agents conservateurs, les pigments et colorants, les émollients, les agents anti-mousse, les huiles ou cires végétales ou animales, les silicones, les parfums, les agents tensioactifs, les plastifiants, les polymères épaississants autres que ceux de l'invention et autres composés similaires.

Bien entendu, l'homme de métier veillera à choisir ce ou ces éventuels composés complémentaires et leur quantité, de manière telle que les propriétés avantageuses attachées intrinsèquement à la composition cosmétique ou dermatologique conforme à l'invention ne soient pas, ou substantiellement pas, altérées par la ou les adjonctions envisagées.

Les compositions cosmétiques ou dermatologiques de la présente invention peuvent se présenter sous une forme fluide, gélifiée, semi-solide, voire solide, par exemple sous forme d'émulsions huile-dans-eau ou eau-dans-huile, de gels aqueux ou hydroalcooliques.

Elles trouvent en particulier une application en tant que produits de soin ou de protection, de produits de maquillage tels que les fonds de teint, rouges à lèvres, fards à joues ou à paupières, mascaras, eye-liners, vernis à ongles, ou de formulations pour masques "peel off".

Une forme de présentation préférée d'une composition selon l'invention est une formulation pour masques "peel off" renfermant l'association d'un poly(alcool vinylique) et d'un dendrimère de type polyester à groupements terminaux hydroxyle.

La présente invention a également pour objet un procédé de traitement cosmétique consistant par exemple
- à appliquer, et éventuellement à faire pénétrer, une composition topique cosmétique selon l'invention sur la peau, les ongles, les fibres kératiniques, les semi-muqueuses et/ou muqueuses, ou
- étaler la composition cosmétique selon la présente invention sur la partie à traiter en une épaisseur permettant la formation d'un film, à laisser la composition en contact avec la peau pendant une durée suffisante pour obtenir l'effet cosmétique désiré ou pour permettre le séchage partiel ou total de la composition et à retire, à la fin de ce temps de pose, par simple pelage le film ainsi formé.

L'invention est illustrée plus en détail dans les exemples suivants.

### Exemple 1

On prépare trois compositions pour masques "peel off" dont deux (compositions **A** et **B**), conformes à la présente invention, comprennent l'association de poly(alcool vinylique) et d'un dendrimère, et une troisième (composition **C**), correspondant à l'état de la technique, renferme, comme seul composant filmogène, du poly(alcool vinylique).

Le tableau ci-dessous regroupe les constituants de ces trois compositions ainsi que les appréciations sensorielles concernant l'élimination par pelage, après séchage, d'un film appliqué sur une plaque de verre.
* le poly(alcool vinylique est le produit AIRVOL 540 commercialisé par la société AIR PRODUCTS et le polyester dendritique est le dendrimère de triméthylolpropane et d'acide diméthylolpropionique à groupements hydroxyle terminaux commercialisé par la société PERSTORP sous la dénomination BOLTORN H40TMP CORE.

On constate que la présence du polymère dendritique renforce la cohésion du film séché et facilite très notablement l'élimination de celui-ci par pelage.

### Exemple 2

### Masque "Peel Off" :

On prépare une formulation pour masques "peel off" en mélangeant les composants suivants en les quantités indiquées :

| | |
|---|---|
| poly(alcool vinylique)* | 10 g |
| polyester dendritique* | 1 g |
| éthanol | 10 g |
| PEG 75 | 1 g |
| glycérol | 2 g |
| principe actif (acides de fruits) | 1 g |
| parfum | 0,4 g |
| conservateurs | 0,3 g |
| eau permutée q. s. | 100 g |

| | |
|---|---|
| * le poly(alcool vinylique) et le polyester dendritique sont ceux utilisés dans l'Exemple 1. | |

### Exemple 3

### Crème de soin filmogène:

On prépare une crème de soin à partir des ingrédients suivants :

| | |
|---|---|
| poly(alcool vinylique)* | 1,5 g |
| polyester dendritique* | 0,25 g |
| éthanol | 2 g |
| stéarate de PEG 40 | 1,2 g |
| glycérol | 3 g |
| stéarate de glycéryle | 1 g |
| alcool cétylique | 2 g |
| cyclométhicone (D₅) | 5 g |
| | huile de karité 5 g |
| principe actif (tocophérol) | 1 g |
| parfums | 0,4 g |
| conservateurs | 0,3 g |
| eau permutée q. s. | 100 g |

| | |
|---|---|
| * le poly(alcool vinylique) et le polyester dendritique sont ceux utilisés dans l'Exemple 1. | |

## Revendications

1. Composition cosmétique ou dermatologique topique susceptible d'être appliquée sur la peau, les fibres kératiniques, les ongles, les semi-muqueuses et/ou les muqueuses, caractérisée par le fait qu'elle comprend, dans un milieu physiologiquement acceptable, un polymère dendritique de type polyester à fonctions hydroxyle terminales.

2. Composition cosmétique ou dermatologique topique susceptible d'être appliquée sur la peau, les fibres kératiniques, les ongles, les semi-muqueuses et/ou les muqueuses, caractérisée par le fait qu'elle comprend, dans un milieu physiologiquement acceptable
(A) un polymère dendritique de type polyester à fonctions hydroxyle terminales, et
(B) au moins un polymère filmogène.

3. Composition selon l'une des revendications 1 et 2,
caractérisée par le fait que le polymère dendritique à fonctions hydroxyle terminales est une macromolécule hautement ramifiée de type polyester, constituée
- d'un motif central dérivé d'un composé initiateur portant une ou plusieurs fonctions hydroxyle (a),
- de motifs d'allongement de chaîne dérivés d'une molécule d'allongement de chaîne portant une fonction carboxyle (b) et au moins deux fonctions hydroxyle (c),
chacune des fonctions hydroxyle (a) de la molécule centrale étant le point de départ d'une réaction de polycondensation (polyestérification) qui débute par la réaction des fonctions hydroxyle (a) de la molécule centrale avec les fonctions carboxyle (b) des molécules d'allongement de la chaîne, puis se poursuit par réaction des fonctions carboxyle (b) avec les fonctions hydroxyle (c) des molécules d'allongement de la chaîne.

4. Composition selon la revendication 3, caractérisée par le fait que le composé initiateur portant une ou plusieurs fonctions hydroxyle formant le motif central est choisi parmi
(a) un alcool monofonctionnel,
(b) un diol aliphatique, cycloaliphatique ou aromatique,
(c) un triol,
(d) un tétrol,
(e) un alcool de sucre,
(f) l'anhydro-ennéa-heptitol ou le dipentaérythritol,
(g) un α-alkylglycoside,
(h) un polymère polyalcoxylé obtenu par polyalcoxylation d'un des alcools (a) à (g), ayant une masse molaire au plus égale à 8000.

5. Composition selon la revendication 4, caractérisée par le fait que le composé initiateur est choisi parmi le ditriméthylolpropane, le ditriméthyloléthane, le dipentaérythritol, le pentaérythritol, un pentaérythritol alcoxylé, le triméthyloléthane, le triméthylolpropane, un triméthylolpropane alcoxylé, le glycérol, le néopentylglycol, le diméthylolpropane ou le 1,3-dioxane-5,5-diméthanol.

6. Composition selon l'une quelconque des revendications 3 à 5, caractérisée par le fait que la molécule d'allongement de chaîne est choisie parmi
- les acides monocarboxyliques comportant au moins deux fonctions hydroxyle,
- les acides monocarboxyliques comportant au moins deux fonctions hydroxyle dont une ou plusieurs porte(nt) un substituant hydroxyalkyle.

7. Composition selon la revendication 6, caractérisée par le fait que la molécule d'allongement de chaîne est choisie parmi l'acide diméthylolpropionique, l'acide α,α-bis(hydroxyméthyl)-butyrique, l'acide α,α,α-tris(hydroxyméthyl)-acétique, l'acide α,α-bis(hydroxyméthyl)-valérique, l'acide α,α-bis(hydroxy)-propionique et l'acide 3,5-dihydroxybenzoïque.

8. Composition selon l'une quelconque des revendications 3 à 7, caractérisée par le fait que le composé initiateur est choisi parmi le ditriméthylolpropane, le triméthylolpropane, un pentaérythritol éthoxylé, le pentaérythritol ou le glycérol, et que la molécule d'allongement de chaîne est l'acide diméthylolpropionique.

9. Composition selon l'une quelconque des revendications 3 à 8, caractérisée par le fait qu'une partie des fonctions hydroxyle terminales du polymère dendritique de type polyester portent des substituants dérivés d'au moins un agent de terminaison de chaîne.

10. Composition selon l'une quelconque des revendications 2 à 9, caractérisée par le fait que le polymère filmogène est un polymère filmogène physiologiquement acceptable choisi parmi les polyuréthannes anioniques, cationiques, non ioniques ou amphotères, les polyuréthannes acryliques, les copolymères de type polyuréthanne-polyvinylpyrrolidone, les poly(ester uréthanne), les poly(éther uréthanne), les polyurées, les poly(urée uréthanne), les polyesters, les poly(ester amide), les polyesters à chaîne(s) grasse(s), les polyamides, les résines époxy-ester, les polymères et/ou copolymères acryliques et/ou vinyliques, les copolymères acryliques/silicones, les copolymères nitrocellulose/acryliques, les polymères d'origine naturelle éventuellement modifiés, les polymères résultant de la polymérisation radicalaire d'un ou de plusieurs monomères à l'intérieur et/ou partiellement en surface de particules préexistantes d'au moins un polymère choisi dans le groupe formé par les polyuréthannes, les polyurées, les polyesters, les poly(ester amide) et/ou les alkydes et des mélanges de ces polymères.

11. Composition selon la revendication 10, caractérisée par le fait que le polymère filmogène est un poly(acétate de vinyle) éventuellement partiellement hydrolysé en poly(alcool vinylique).

12. Composition selon l'une des revendications 10 et 11, caractérisée par le fait que le polymère filmogène est le poly(alcool vinylique).

13. Composition selon l'une quelconque des revendications 2 à 12, caractérisée par le fait que le rapport en poids du polymère dendritique au polymère filmogène est compris dans l'intervalle allant de 1/100 à 1/1.

14. Composition selon l'une quelconque des revendications 1 à 13, caractérisée par le fait que la concentration du polymère dendritique rapportée à la composition totale est comprise dans l'intervalle allant de 0,1 à 5 % en poids.

15. Composition selon l'une quelconque des revendications 1 à 14, caractérisée par le fait qu'elle contient en outre un ou plusieurs principes actifs cosmétiques ou pharmaceutiques utilisés habituellement en application topique.

16. Composition cosmétique ou dermatologique selon la revendication 15, caractérisée par le fait que le ou les principes actifs sont choisis parmi les anti-inflammatoires, les anti-bactériens, les anti-fongiques, les anti-viraux, les anti-séborrhéiques, les anti-acnéiques, les kératolytiques, les antihistaminiques, les anesthésiques, les agents cicatrisants, les modificateurs de la pigmentation, les filtres solaires, les agents anti-radicaux libres, les agents hydratants, les vitamines, les protéines, les céramides et autres composés similaires.

17. Composition selon l'une quelconque des revendications 1 à 16, caractérisée en ce qu'elle contient en outre un ou plusieurs solvants ou adjuvants choisis parmi l'eau, les monoalcools inférieurs tels que l'éthanol ou l'isopropanol, les glycols tels que le diéthylèneglycol, les éthers de glycols tels que les alkyléthers d'éthylèneglycol ou de diéthylèneglycol, les esters d'acides gras, les agents régulateurs de pH, les anti-oxydants, les agents conservateurs, les pigments et colorants, les émollients, les agents anti-mousse, les huiles ou cires végétales ou animales, les silicones, les parfums, les agents tensioactifs, les plastifiants, les polymères épaississants autres que ceux de l'invention et autres composés similaires, et les mélanges de ces composés.

18. Composition cosmétique ou dermatologique selon l'une quelconque des revendications 1 à 17, caractérisée par le fait qu'elle se présente sous forme d'une crème de soin ou de protection, d'un produit de maquillage tel que les fonds de teint, rouges à lèvres, fards à joues ou à paupières, mascaras, eye-liners, vernis à ongles, ou d'une formulation pour masques "peel off".

19. Procédé de traitement cosmétique caractérisé par le fait qu'il consiste à appliquer une composition selon l'une quelconque des revendications 1 à 18 sur la peau, les ongles, les fibres kératiniques, les semi-muqueuses et/ou muqueuses, les méthodes de traitement thérapeutiques étant exclues.

20. Procédé de traitement cosmétique, caractérisé par le fait que l'on étale la composition selon l'une quelconque des revendications 2 à 18 sur la partie à traiter en une épaisseur permettant la formation d'un film, que l'on laisse la composition en contact avec la peau pendant une durée suffisante pour obtenir l'effet cosmétique désiré ou pour permettre le séchage partiel ou total de la composition et que l'on retire, à la fin de ce temps de pose, par simple pelage le film ainsi formé, les méthodes de traitement thérapeutiques étant exclues.

21. Utilisation de la composition selon l'une quelconque des revendications 1 à 18 pour la préparation d'une composition dermatologique destinée au traitement de la peau.

## Claims

1. Topical cosmetic or dermatological composition capable of being applied to the skin, the keratinous fibres, the nails, the semimucous membranes and/or the mucous membranes, characterized in that it comprises, in a physiologically acceptable medium, a dendritic polymer of the polyester type with terminal hydroxyl functions.

2. Topical cosmetic or dermatological composition capable of being applied to the skin, the keratinous fibres, the nails, the semimucous membranes and/or the mucous membranes, characterized in that it comprises, in a physiologically acceptable medium
(A) a dendritic polymer of the polyester type with terminal hydroxyl functions, and
(B) at least one film-forming polymer.

3. Composition according to either of Claims 1 and 2, characterized in that the dendritic polymer with terminal hydroxyl functions is a highly branched macromolecule of the polyester type, consisting
- of a central unit derived from an initiator compound carrying one or more hydroxyl functions (a),
- chain-extension units derived from a chain-extension molecule carrying a carboxyl function (b) and at least two hydroxyl functions (c),
each of the hydroxyl functions (a) of the central molecule being the starting point of a polycondensation reaction (polyesterification) which starts with the reaction of the hydroxyl functions (a) of the central molecule with the carboxyl functions (b) of the chain-extension molecules, and then continues with the reaction of the carboxyl functions (b) with the hydroxyl functions (c) of the chain-extension molecules.

4. Composition according to Claim 3, characterized in that the initiator compound carrying one or more hydroxyl functions forming the central unit is chosen from
(a) a monofunctional alcohol,
(b) an aliphatic, cycloaliphatic or aromatic diol,
(c) a triol,
(d) a tetrol,
(e) a sugar alcohol,
(f) anhydro-ennea-heptitol or dipentaerythritol,
(g) an α-alkylglycoside,
(h) a polyalkoxylated polymer obtained by polyalkoxylation of one of the alcohols (a) to (g), having a molar mass at most equal to 8000.

5. Composition according to Claim 4, characterized in that the initiator compound is chosen from ditrimethylolpropane, ditrimethylolethane, dipentaerythritol, pentaerythritol, an alkoxylated pentaerythritol, trimethylolethane, trimethylolpropane, an alkoxylated trimethylolpropane, glycerol, neopentyl glycol, dimethylolpropane or 1,3-dioxane-5,5-dimethanol.

6. Composition according to any one of Claims 3 to 5, characterized in that the chain-extension molecule is chosen from
- monocarboxylic acids containing at least two hydroxyl functions, and
- monocarboxylic acids containing at least two hydroxyl functions of which one or more carry a hydroxyalkyl substituent.

7. Composition according to Claim 6, characterized in that the chain-extension molecule is chosen from dimethylolpropionic acid, α,α-bis(hydroxymethyl) butyric acid, α,α,α-tris(hydroxymethyl)acetic acid, α,α-bis(hydroxymethyl)valeric acid, α,α-bis(hydroxy)propionic acid and 3,5-dihydroxybenzoic acid.

8. Composition according to any one of Claims 3 to 7, characterized in that the initiator compound is chosen from ditrimethylolpropane, trimethylolpropane, an ethoxylated pentaerythritol, pentaerythritol or glycerol, and in that the chain-extension molecule is dimethylolpropionic acid.

9. Composition according to any one of Claims 3 to 8, characterized in that some of the terminal hydroxyl functions of the polyester-type dendritic polymer carry substituents derived from at least one chain-terminating agent.

10. Composition according to any one of Claims 2 to 9, characterized in that the film-forming polymer is a physiologically acceptable film-forming polymer chosen from anionic, cationic, nonionic or amphoteric polyurethanes, acrylic polyurethanes, copolymers of the polyurethane-pclyvinylpyrrolidone type, poly(urethane esters), poly(urethane ethers), polyureas, poly(urethane ureas), polyesters, poly(amide esters), polyesters with fatty chain(s), polyamides, epoxy ester resins, acrylic and/or vinyl polymers and/or copolymers, acrylic/silicone copolymers, nitrocellulose/acrylic copolymers, optionally modified polymers of natural origin, polymers resulting from the free-radical polymerization of one or more monomers inside and/or partially at the surface of pre-existing particles of at least one polymer chosen from the group formed by polyurethanes, polyureas, polyesters, poly(amide esters) and/or alkyds and mixtures of these polymers.

11. Composition according to Claim 10, characterized in that the film-forming polymer is a poly(vinyl acetate) optionally partially hydrolysed to poly(vinyl alcchol).

12. Composition according to either of Claims 10 and 11, characterized in that the film-forming polymer is poly(vinyl alcohol).

13. Composition according to any one of Claims 2 to 12, characterized in that the weight ratio of the dendritic polymer to the film-forming polymer is within the interval ranging from 1/100 to 1/1.

14. Composition according to any one of Claims 1 to 13, characterized in that the concentration of the dendritic polymer relative to the total composition is within the interval ranging from 0.1 to 5% by weight.

15. Composition according to any one of Claims 1 to 14, characterized in that it contains, in addition, one or more cosmetic or pharmaceutical active ingredients customarily used in topical application.

16. Cosmetic or dermatological composition according to Claim 15, characterized in that the active ingredient(s) are chosen from anti-inflammatory agents, antibacterials, antifungals, antivirals, antiseborrhoeic agents, anti-acne agents, keratolytics, antihistamines, anaesthetics, cicatrizing agents, pigmentation modifiers, sunscreens, anti-free radical agents, moisturizing agents, vitamins, proteins, ceramides and other similar compounds.

17. Composition according to any one of Claims 1 to 16, characterized in that it contains, in addition, one or more solvents or adjuvants chosen from water, lower monoalcohols such as ethanol or isopropanol, glycols such as diethylene glycol, glycol ethers such as alkyl ethers of ethylene glycol or of diethylene glycol, or fatty acid esters, pH-regulating agents, antioxidants, preservatives, pigments and colourings, emollients, antifoams, plant or animal oils or waxes, silicones, perfumes, surfactants, plasticizers, thickening polymers other than those of the invention and other similar compounds, and mixtures of these compounds.

18. Cosmetic or dermatological composition according to any one of Claims 1 to 17, characterized in that it is provided in the form of a care or protection cream, a make-up product such as foundations, lipsticks, blushers or eyeshadows, mascaras, eyeliners, nail varnish, or a formulation for "peel off" masks.

19. Method of cosmetic treatment, characterized in that it consists in applying a composition according to any one of Claims 1 to 18 to the skin, the nails, the keratinous fibres, the semimucous membranes and/or the mucous membranes, therapeutic methods of treatment being excluded.

20. Method of cosmetic treatment, characterized in that the composition according to any one of Claims 2 to 18 is spread on the part to be treated in a thickness allowing the formation of a film, in that the composition is left: in contact with the skin for a sufficient period to obtain the desired cosmetic effect or to allow the partial or complete drying of the composition and in that the film thus formed is removed simply by peeling off at the end of this exposure time, therapeutic methods of treatment being excluded.

21. Use of the composition according to any one of Claims 1 to 18 for the preparation of a dermatological composition intended for the treatment of the skin.

## Patentansprüche

1. Kosmetische oder dermatologische topische Zusammensetzung zum Aufbringen auf die Haut, auf Keratinfasern, Nägel, Semischleimhäute und/oder Schleimhäute, dadurch gekennzeichnet, daß sie in einem physiologisch akzeptablem Medium ein dendritisches Polymer vom Polyestertyp mit endständigen Hydroxylfünktionen enthält.

2. Kosmetische oder dermatologische topische Zusammensetzung zum Auftragen auf die Haut, auf Keratinfasern, Nägel, Semischleimhäute und/oder Schleimhäute, dadurch gekennzeichnet, daß sie in einem physiologisch akzeptablen Medium enthält:
(A) ein dendritisches Polymer vom Polyestertyp mit endständigen Hydroxylfunktionen und
(B) mindestens ein filmbildendes Polymer.

3. Zusammensetzung nach Anspruch 1 oder 2, dadurch gekennzeichnet, daß das dendritische Polymer mit endständigen Hydroxylfunktionen ein hochverzweigtes Makromolekül vom Polyestertyp ist, das besteht aus
- einer zentralen Einheit, die von einer Initiatorverbindung mit einer oder mehreren Hydroxylfunktionen (a) abgeleitet ist,
- Kettenverlängerungseinheiten, die von einem Kettenverlängerungsmolekül mit einer Carboxylfunktion (b) und mindestens zwei Hydroxylfunktionen (c) abgeleitet sind,
wobei jede der Hydroxylfunktionen (a) des zentralen Moleküls der Ausgangspunkt für eine Polykondensationsreaktion (Polyesterbildung) ist, die durch Umsetzung der Hydroxylfunktionen (a) des zentralen Moleküls mit den Carboxylfunktionen (b) der Kettenverlängerungsmoleküle beginnt und sich durch Umsetzung der Carboxylfunktionen (b) mit den Hydroxylfunktionen (c) der Kettenverlängerungsmoleküle fortsetzt.

4. Zusammensetzung nach Anspruch 3, dadurch gekennzeichnet, daß die Initiatorverbindung, die eine oder mehrere Hydroxylfunktionen trägt und die zentrale Einheit bildet, ausgewählt ist unter
(a) einem einwertigen Alkohol,
(b) einem aliphatischen, cycloaliphatischen oder aromatischen Diol,
(c) einem Triol,
(d) einem Tetrol,
(e) einem Zuckeralkohol,
(f) Anhydroenneaheptitol oder Dipentaerythrit,
(g) einem α-Alkylglycosid,
(h) einem durch Polyalkoxylierung eines der Alkohole (a) bis (g) erhaltenen polyalkoxylierten Polymer mit einer Molmasse von höchstens 8000.

5. Zusammensetzung nach Anspruch 4, dadurch gekennzeichnet, daß die Initiatorverbindung ausgewählt ist unter Ditrimethylolpropan, Ditrimethylolethan, Dipentaerythrit, Pentaerythrit, einem alkoxylierten Pentaerythrit, Trimethylolethan, Trimethylolpropan, einem alkoxylierten Trimethylolpropan, Glycerin, Neopentylglykol, Dimethylolpropan oder 1,3-Dioxan-5,5-dimethanol.

6. Zusammensetzung nach einem der Ansprüche 3 bis 5, dadurch gekennzeichnet, daß das Kettenverlängerungsmolekül ausgewählt ist unter
- Monocarbonsäuren mit mindestens zwei Hydroxylfunktionen,
- Monocarbonsäuren mit mindestens zwei Hydroxylfunktionen, von denen eine oder mehrere einen Hydroxyalkylsubstituenten aufweisen.

7. Zusammensetzung nach Anspruch 6, dadurch gekennzeichnet, daß das Kettenverlängerungsmolekül ausgewählt ist unter Dimethylolpropionsäure, α,α-Bis(hydroxymethyl)-buttersäure, α,α,α Tris (hydroxymethyl)-essigsäure, α,α Bis(hydroxymethyl)-valeriansäure, α,α Bis(hydroxy)-propionsäure und 3,5-Dihydroxybenzoesäure.

8. Zusammensetzung nach einem der Ansprüche 3 bis 7, dadurch gekennzeichnet, daß die Initiatorverbindung ausgewählt ist unter Ditrimethylolpropan, Trimethylolpropan, einem ethoxylierten Pentaerythrit, Pentaerythrit oder Glycerin und das Kettenverlängerungsmolekül Dimethylolpropionsäure ist.

9. Zusammensetzung nach einem der Ansprüche 3 bis 8, dadurch gekennzeichnet, daß ein Teil der endständigen Hydroxylfunktionen des dendritischen Polymers vom Polyestertyp Substituenten tragen, die von mindestens einem Kettenabbruchmittel abgeleitet sind.

10. Zusammensetzung nach einem der Ansprüche 2 bis 9, dadurch gekennzeichnet, daß das filmbildende Polymer ein physiologisch akzeptables filmbildendes Polymer ist, das ausgewählt ist unter anionischen, kationischen, nichtionischen oder amphoteren Polyurethanen, Acrylpolyurethanen, Copolymeren vom Polyurethan-Polyvinylpyrrolidon-Typ, Poly(esterurethanen), Poly(etherurethanen), Polyharnstoffen, Poly(hamstoffurethanen), Polyestern, Poly(esteramiden), Polyestern mit Fettsäurekette(n), Polyamiden, Epoxy-Ester-Harzen, Acryl- und/oder Vinyl-Polymeren und/oder -Copolymeren, Acryl/Silicon-Copolymeren, Nitrocellulose/Acryl-Copolymeren, Polymeren natürlichen Ursprungs, die gegebenenfalls modifiziert sind, und Polymeren, die aus der radikalischen Polymerisation eines oder mehrerer Monomerer im Inneren und/oder teilweise an der Oberfläche präexistierender Partikel aus mindestens einem Polymer resultieren, das ausgewählt ist unter Polyurethanen, Polyharnstoffen, Polyestern, Poly(esteramiden) und/oder Alkydharzen, sowie Gemischen dieser Polymeren.

11. Zusammensetzung nach Anspruch 10, dadurch gekennzeichnet, daß das filmbildende Polymer ein Poly(vinylacetat) ist, das gegebenenfalls partiell zu Poly(vinylalkohol) hydrolysiert ist.

12. Zusammensetzung nach Anspruch 10 oder 11, dadurch gekennzeichnet, daß das filmbildende Polymer Poly(vinylalkohol) ist.

13. Zusammensetzung nach einem der Ansprüche 2 bis 12, dadurch gekennzeichnet, daß das Gewichtsverhältnis des dendritischen Polymers zum filmbildenden Polymer im Bereich von 1/100 bis 1/1 liegt.

14. Zusammensetzung nach einem der Ansprüche 1 bis 13, dadurch gekennzeichnet, daß die Konzentration des dendritischen Polymers, bezogen auf die gesamte Zusammensetzung, im Bereich von 0,1 bis 5 Gew.-% liegt.

15. Zusammensetzung nach einem der Ansprüche 1 bis 14, dadurch gekennzeichnet, daß sie ferner einen oder mehrere kosmetische oder pharmazeutische Wirkstoffe enthält, die üblicherweise zur topischen Anwendung verwendet werden.

16. Kosmetische oder dermatologische Zusammensetzung nach Anspruch 15, dadurch gekennzeichnet, daß der oder die Wirkstoffe ausgewählt sind unter entzündungshemmenden Mitteln, antibakteriellen Mitteln, Mitteln gegen Pilze, antiviralen Mitteln, antiseborrhoischen Mitteln, Antiaknemitteln, Keratolytika, Antihistaminika, Anästhetika, narbenbildenden Mitteln, Mitteln zur Modifizierung der Pigmentierung, Sonnenfiltern, Mitteln gegen freie Radikale, Hydratisierungsmitteln, Vitaminen, Proteinen, Ceramiden und anderen, ähnlichen Verbindungen.

17. Zusammensetzung nach einem der Ansprüche 1 bis 16, dadurch gekennzeichnet, daß sie ferner ein oder mehrere Lösungsmittel oder Adjuvantien enthält, die ausgewählt sind unter Wasser, niederen einwertigen Alkoholen wie Ethanol oder Isopropanol, Glykolen wie Diethylenglykol, Glykolethern wie Ethylenglykolalkylethern oder Diethylenglykolalkylethern, Fettsäureestern, Mitteln zur pH-Regulierung, Antioxidantien, Konservierungsmitteln, Pigmenten und Färbemitteln, Emollientien, Antischaummitteln, pflanzlichen oder tierischen Ölen oder Wachsen, Siliconen, Parfums, grenzflächenaktiven Mitteln, Plastifizierungsmitteln, Polymeren mit Verdickungswirkung, die von den Polymeren der Erfindung verschieden sind, sowie anderen, ähnlichen Verbindungen und Gemischen dieser Verbindungen.

18. Kosmetische oder dermatologische Zusammensetzung nach einem der Ansprüche 1 bis 17, dadurch gekennzeichnet, daß sie als Pflege- oder Schutzcreme, Produkt zum Schminken wie zum Beispiel als Make-up, Lippenstift, Wangenrouge oder Lidschatten, Mascara, Eyeliner, Nagellack oder als Peel-off-Maske vorliegt.

19. Verfahren zur kosmetischen Behandlung, dadurch gekennzeichnet, daß es im Aufbringen einer Zusammensetzung nach einem der Ansprüche 1 bis 18 auf die Haut, die Nägel, Keratinfasern, Semischleimhäute und/oder Schleimhäute besteht, wobei Verfahren zur therapeutischen Behandlung ausgeschlossen sind.

20. Verfahren zur kosmetischen Behandlung, gekennzeichnet durch Auftragen der Zusammensetzung nach einem der Ansprüche 2 bis 18 auf den zu behandelnden Bereich in einer Dicke, welche die Bildung eines Films erlaubt, Belassen der Zusammensetzung im Kontakt mit der Haut während einer Zeit, die ausreicht, um die erwünschte kosmetische Wirkung zu erzielen oder ein teilweises oder vollständiges Trocknen der Zusammensetzung zu erlauben, und Abnehmen des so gebildeten Films am Ende dieser Einwirkungsdauer durch einfaches Abziehen, wobei Verfahren zur therapeutischen Behandlung ausgeschlossen sind.

21. Verwendung der Zusammensetzung nach einem der Ansprüche 1 bis 18 zur Herstellung einer dermatologischen Zusammensetzung, die zur Behandlung der Haut vorgesehen ist.
